# FASCICULE DE BREVET EUROPEEN

(11) **EP 1 233 787 B1**
(45) Date de publication et mention de la délivrance du brevet: **08.12.2004**
(21) Numéro de dépôt: 00976116.4
(22) Date de dépôt: 08.11.2000
(51) Int. Cl.: A61K 45/06, A61K 31/495, A61K 31/425, A61K 31/42, A61K 31/415, A61K 31/55, A61K 31/47, A61P 35/00

(54) **PRODUIT COMPRENANT UN INHIBITEUR DE LA TRANSDUCTION DES SIGNAUX DES PROTEINES G HETEROTRIMERIQUES EN ASSOCIATION AVEC UN AUTRE AGENT ANTI-CANCEREUX POUR UNE UTILISATION THERAPEUTIQUE DANS LE TRAITEMENT DU CANCER**
ZUSAMMENSETZUNG ENTHALTEND EINEN INHIBITOR DER SIGNALTRANSDUKTION VON HETEROTRIMERISCHEN G-PROTEINEN IN KOMBINATION MIT EINEM ANDEREN ANTI-KREBS MITTEL ZU EINER THERAPEUTISCHEN VERWENDUNG IN DER KREBSBEHANDLUNG
PRODUCT INHIBITING TRANSDUCTION OF G HETEROTRIMERIC PROTEIN SIGNALS COMBINED WITH ANOTHER ANTI-CANCER AGENT FOR THERAPEUTIC USE IN CANCER TREATMENT

(30) Priorité: 09.11.1999 FR 9914037; 06.01.2000 FR 0000104
(43) Date de publication de la demande: 28.08.2002
(60) Demande divisionnaire: 04075491.3
(73) Titulaire: SOCIETE DE CONSEILS DE RECHERCHES ET D'APPLICATIONS SCIENTIFIQUES S.A. (S.C.R.A.S.), 75016 Paris (FR)
(72) Inventeur: PREVOST, Grégoire, F-92160 Antony (FR); LONCHAMPT, Marie-Odile, F-94550 Chevilly-Larue (FR); GORDON, Thomas, Medway, MA 02053 (US); MORGAN, Barry, Franklin, MA 02038 (US)
(74) Mandataire: Bourgouin, André
(86) Numéro de dépôt international: PCT/FR2000/003098
(87) Numéro de publication internationale: WO 2001/034203

(56) Documents cités:
- WO-A-97/30053

## Description

La présente invention concerne un produit comprenant au moins un inhibiteur de la transduction des signaux des protéines G hétérotrimériques, en association avec au moins un autre agent anti-cancéreux choisi parmi le groupe composé du taxol, de la gemcitabine et des inhibiteurs de prényltransférases, pour une utilisation thérapeutique simultanée, séparée ou étalée dans le temps, dans le traitement du cancer.

Le développement des nouveaux traitements anti-cancéreux passent en grande partie par la découverte d'associations efficaces entre différentes classes thérapeutiques pour accentuer l'effet antitumoral de chaque classe.

L'association entre l'anticorps anti-Her-2/ncu et le cis-platine ou l'étoposide inhibe la prolifération des cellules tumorales mammaires de manière plus importante que la simple addition des effets de chaque produit (cf. Pegram, M., et coll., *Oncogene*, **18**(1999): 2241-2251). L'association de cet anticorps avec le taxol ou le méthotrexate montre une addition des effets alors que son association avec le 5-fluorouracyle montre un antagonisme des produits (McGuire W.P. et coll., *Semin*. *Oncol*. **1997** Feb. 24 (1 Suppl 2):S2-13-S2-16).

Les inhibiteurs de farnésyltransférases agissent en synergie avec des agents qui dépolymérisent les microtubules (taxol, épothilones) (cf. Moasser et coll., *Proc. Natl*. *Acad. Sci*. *U*.*S*.*A*. (1998), **95**, 1369-1374). Les associations d'inhibiteurs de farnésyltransférases avec les cytotoxiques doxorubicine, cisplatine ou 5-fluorouracyle montrent seulement une addition des effets.

Les protéines G hétérotrimériques sont, en fait, l'association structurale de trois sous-unités distinctes appelées α, β et γ, mais fonctionnent comme des entités dissociables constituées par des sous-unités α d'un côté et des dimères β/γ de l'autre. Différentes formes de sous-unités de type α, β et γ sont décrites.

Les protéines G participent à la transmission de signaux de l'extérieur de la cellule grâce à son interaction avec les récepteurs à sept domaines transmembranaires vers l'intérieur par l'intermédiaire de différents effecteurs incluant l'adénylate cyclase, la phospholipase C ou encore les canaux ioniques. L'enzyme adénylate cyclase génère de l'adénosine monophosphate cyclique (AMPc) (cf. Gilman, *Biosci. Rep.* (1995), **15**, 65-97). Ainsi, on sait que pour activer l'adénylate cyclase, il est nécessaire que les protéines G soient transitoirement dans une forme hétérotrimérique, forme dans laquelle le monomère constitué par une sous-unité α est associé au dimère constitué par les sous-unités β et γ. On sait encore que pour que les protéine G se trouvent dans leur forme hétérotrimérique, il faut qu'elles soient fixées par leurs sous-unités γ à la membrane. C'est uniquement dans cette situation que le signal de l'extérieur de la cellule peut activer la sous-unité α d'une protéine G, laquelle pourra, après dissociation, moduler les effecteurs comme l'adénylate cyclase et moduler la production d'AMPc.

On sait aussi que les dimères β/γ peuvent activer directement des effecteurs conduisant à l'activation de kinases régulées par des signaux extracellulaires (ERKs) ou des MAP kinases. Un lien direct entre les sous-unités β/γ et les kinases src ou src like a été démontré (cf. Gutkind, *J*. *Biol*. *Chem*. (1998), **273**, 1839-1842).

Les effets néfastes d'un taux anormal d'AMPc sont également connus et ont notamment lieu au niveau des fonctions biologiques ou désordres suivants : odorat, goût, perception de la lumière, neurotransmission, neurodégénérescence, fonctionnement des glandes endocrines et exocrines, régulations autocrine et paracrine, tension artérielle, embryogénèse, prolifération cellulaire bénigne, oncogénèse, infection virale et fonctions immunologiques, diabète et obésité.

La demanderesse avait elle-même déjà décrit dans la demande de brevet PCT WO 00/02881 l'utilisation des composés de formule générale **(I)** en tant qu'inhibiteurs de protéine G. Certains de ces produits avaient été décrits auparavant dans la demande de brevet PCT WO 97/30053.

Les inhibiteurs de prényltransférases sont déjà utilisés dans le domaine du traitement du cancer (cf. Sebti et coll., *Pharmacol*. *Ther*. (1997), **74**, 103-114; Sepp-Lorenzino et coll., *Cancer Res.* (1997), **55**, 5302-5309). L'utilité des inhibiteurs de prényltransférases dans ce type de traitement proviendrait de leur action qui empêcherait la prénylation au niveau du substrat Ras. Cependant, la prénylation de certaines formes de Ras n'est pas modifiée par les inhibiteurs de prénylation (Lerner et coll., *Oncogene* (1997), **15**, 1283-1288).

En ce qui concerne les agents anti-cancéreux, des inhibiteurs de prényltransférases sont notamment décrits dans les demandes de brevet suivantes : demandes PCT WO 97/21701, WO 97/16443, WO 98/00409, WO 96/21456, WO 97/24378, WO 97/17321, WO 97/18813, WO 95/00497 ; brevets US 5,532,359, US 5,523,430, US 5,510,510 et US 5,627,202. Par ailleurs, les composés de formule générale **(II)** ont été décrits dans la demande de brevet PCT WO 00/39130. Le taxol est quant à lui notamment décrit dans *Merck Index,* 11th ed., 1989, sous le numéro de rubrique 9049 et dans les références citées. Des analogues de camptothécines ont notamment été décrits dans le brevet US 4,894,456 et dans les demandes de brevet PCT WO 94/11376, WO 97/00876, WO 98/28304, WO 98/28305, WO 99/11646 et WO 99/33829.

Un produit selon l'invention offre l'avantage de pouvoir utiliser des doses moins élevées des agents anti-cancéreux choisis, ce qui a pour principal effet de diminuer la toxicité du traitement tout en obtenant un effet pharmacologique au minimum additif.

L'invention a donc pour objet un produit comprenant au moins un inhibiteur de la transduction des signaux des protéines G hétérotrimériques en association avec au moins un agent anti-cancéreux choisi parmi le groupe composé du taxol, de la gemcitabine et des inhibiteurs de prényltransférases, pour une utilisation thérapeutique simultanée, séparée ou étalée dans le temps, dans le traitement du cancer.

De préférence, l'inhibiteur de prényltransférases associé à l'inhibiteur de la transduction des signaux des protéines G hétérotrimériques sera un inhibiteur de farnésyltransférases.

Bien que le taxol, les analogues du taxol, la gemcitabine et les inhibiteurs de prényltransférases, soient préférés, de nombreux autres agents anti-cancéreux peuvent être également associés, à un inhibiteur de la transduction des signaux des protéines G hétérotrimériques, par exemple : des inhibiteurs enzymatiques comme les inhibiteurs de topoisomérases comme la campthotécine et les analogues de la camptothécine (sous forme d'analogues comportant un cycle lactonique E à six chaînons tels par exemple les composés décrits dans la demande de brevet PCT WO 94/11376, sous forme d'analogues comportant un cycle lactonique E à sept chaînons tels par exemple les composés décrits dans la demande de brevet PCT WO 97/00876 ou encore sous forme d'analogues tétracycliques ouverts tels par exemple les composés décrits dans la demande de brevet PCT WO 99/33829), les inhibiteurs de phosphatases Cdc25, les inhibiteurs de MAP kinases ou de MAP kinases kinases, les inhibiteurs de protéine kinase C, les inhibiteurs de tyrosine kinases, les inhibiteurs de télomérases ; des inducteurs d'apoptose ; des agents alkylants comme le cis-platine ; des agents anti-métaboliques comme le 5-fluorouracile ; des agents de différentiation ; des poisons du fuseau cellulaire ; des inhibiteurs d'angiogénèse ; des anti-hormones ou des antagonistes des récepteurs stéroïdiens ; des anti-oxydants ; des agents anti-sens ; des agents anti-p53 (thérapie génique) ; des agents de chémoprévention ; des agents anti-viraux ; des agents immunothérapeutiques ; des anti-corps comme l'héréguline.

L'inhibiteur de la transduction des signaux des protéines G hétérotrimériques est choisi parmi :
- la 7-(2-amino-1-oxo-3-thiopropyl)-8-(cyclohexylméthyl)-2-(2-méthylphényl)-5,6,7,8-tétrahydroimidazo[1,2a]pyrazine ;
- la 7-(2-amino-1-oxo-3-thiopropyl)-8-(cyclohexylméthyl)-2-phényl-5,6,7,8-tétrahydroimidazo[1,2a]pyrazine ;
ou un sel pharmaceutiquement acceptable d'un de ces derniers.

De préférence, lorsque l'agent anti-cancéreux associé à l'inhibiteur de la transduction des signaux des protéines G hétérotrimériques sera un inhibiteur de prényltransférases, il s'agira d'un inhibiteur de farnésyltransférases.

Selon l'invention, les inhibiteurs de la transduction des signaux des protéines G hétérotrimériques seront choisis parmi le groupe composé :
- de la 7-(2-amino-1-oxo-3-thiopropyl)-8-(cyclohexylméthyl)-2-(2-methylphényl)-5,6,7,8-tétrahydroimidazo[1,2a]pyrazine ; et
- de la 7-(2-amino-1-oxo-3-thiopropyl)-8-(cyclohexylméthyl)-2-phényl-5,6,7,8-tétrahydroimidazo[1,2a]pyrazine ;
et des sels pharmaceutiquement acceptables de ces derniers.

Toujours selon l'invention, les agents anti-cancéreux associés auxdits inhibiteurs de la transduction des signaux des protéines G hétérotrimériques seront choisis parmi le groupe composé :
- de la 1-(2-(1-((4-cyano)phénylméthyl)imidazol-4-yl)-1-oxoéthyl)-2,5-dihydro-4-(2-méthoxyphényl)imidazo[1,2c][1,4]benzodiazépine ;
- de la 4-(2-bromophényl)-1-(2-(1-((4-cyano-3-méthoxy)phénylméthyl)-imidazo-5-yl)-1-oxoéthyl)-1,2-dihydro-8-fluoro-imidazo[1,2a][1,4]-benzodiazépine ;
- de la (±)-4-(3-chlorophényl)-6-[(4-chlorophényl)-amino-(1-méthyl-1H-imidazol-5-yl)méthyl]-1-méthyl-2(1H)quinolinone ;
- du taxol ;
- de la gemcitabine ;
et des sels pharmaceutiquement acceptables de ces derniers.

Optionnellement, on pourra également faire entrer un composé anti-cancéreux supplémentaire, distinct de l'agent anti-cancéreux associé à l'inhibiteur de la transduction des signaux des protéines G hétérotrimériques, dans la composition du produit de l'invention. De préférence, ledit composé supplémentaire sera choisi parmi le groupe composé :
- de la 1-(2-(1-((4-cyano)phénylméthyl)imidazol-4-yl)-1-oxoéthyl)-2,5-dihydro-4-(2-méthoxyphényl)imidazo[1,2c][1,4]benzodiazépine ;
- de la 4-(2-bromophényl)-1-(2-(1-((4-cyano-3-méthoxy)phénylméthyl)-imidazo-5-yl)-1-oxoéthyl)-1,2-dihydro-8-fluoro-imidazo[1,2a][1,4]-benzodiazépine
- de la (±)-4-(3-chlorophényl)-6-[(4-chlorophényl)-amino-(1-méthyl-1H-imidazol-5-yl)méthyl]-1-méthyl-2(1H)quinolinone ;
- du taxol ;
- de la gemcitabine ;
et des sels pharmaceutiquement acceptables de ces derniers.

L'invention a également pour objet une composition pharmaceutique comprenant au moins un des produits selon l'invention.

Les compositions pharmaceutiques comprenant un produit selon l'invention peuvent être sous forme de solides, par exemple des poudres, des granules, des comprimés, des gélules, des liposomes ou des suppositoires. Les supports solides appropriés peuvent être, par exemple, le phosphate de calcium, le stéarate de magnésium, le talc, les sucres, le lactose, la dextrine, l'amidon, la gélatine, la cellulose, la cellulose de méthyle, la cellulose carboxyméthyle de sodium, la polyvinylpyrrolidine et la cire.

Les compositions pharmaceutiques comprenant un produit selon l'invention peuvent aussi se présenter sous forme liquide, par exemple, des solutions, des émulsions, des suspensions ou des sirops. Les supports liquides appropriés peuvent être, par exemple, l'eau, les solvants organiques tels que le glycérol ou les glycols, de même que leurs mélanges, dans des proportions variées, dans l'eau.

L'administration d'un médicament selon l'invention pourra se faire par voie topique, orale, parentérale, par injection (intramusculaire, sous-cutanée, intraveineuse, etc.), etc. La voie d'administration dépendra bien entendu du type de maladie à traiter.

Les doses d'administration suivantes (journalières, sauf indication contraire) pourront être envisagées pour les différents composés entrant dans la composition d'un produit selon l'invention :
- Inhibiteur de la transduction des signaux des proteines G hétérotrimériques : de 50 à 200 mg/m² par voie intrapéritonéale ;
- Inhibiteur de farnésyltransférase : de 50 à 500 mg/m² per os ;
- taxol : de 1 à 10 mg/kg (voie intrapéritonéale) ou 1 à 3 mg/kg (voie intraveineuse) ;
- gemcitabine : de 100 à 500 mg/m² par voie intraveineuse (perfusions de 6 h environ).

### Préparation de certains composés entrant dans la composition des produits de l'invention :

**I)** Les inhibiteurs de la transduction des signaux des protéines G hétérotrimériques sont préparés selon des méthodes analogues à celles décrites dans la demande de brevet PCT WO 97/30053.
   Toutefois, les composés suivants ont été décrits ultérieurement dans la demande de brevet WO 00/02881 :
   - la 7-(2-amino-1-oxo-3-thiopropyl)-8-(cyclohexylméthyl)-2-(2-méthylphényl)-5,6,7,8-tétrahydroimidazo[1,2a]pyrazine ; et
   - la 7-(2-amino-1-oxo-3-thiopropyl)-8-(cyclohexylméthyl)-2-phényl-5,6,7,8-tétrahydroimidazo[1,2a]pyrazine.
**II)** Les composés 1-(2-(1-((4-cyano)phénylméthyl)imidazol-4-yl)-1-oxoéthyl)-2,5-dihydro-4-(2-méthoxyphényl)imidazo[1,2c][1,4]benzodiazépine et la 4-(2-bromophényl)-1-(2-( 1-((4-cyano-3-méthoxy)phénylméthyl)imidazo-5-yl)-1-oxoéthyl)-1,2-dihydro-8-fluoroimidazol[1,2a][1,4]-benzodiazépine, sont préparés selon les procédures décrites dans la demande de brevet PCT WO 00/39130.

**C)** D'autres composés sont préparés selon les méthodes décrites dans la demande de brevet PCT WO 97/21701.

A moins qu'ils ne soient définis d'une autre manière, tous les termes techniques et scientifiques utilisés ici ont la même signification que celle couramment comprise par un spécialiste ordinaire du domaine auquel appartient cette invention.

Les exemples suivants sont présentés pour illustrer les procédures ci-dessus et ne doivent en aucun cas être considérés comme une limite à la portée de l'invention.

### EXEMPLES

Afin d'illustrer l'utilité de l'invention, on étudiera l'effet d'un traitement sur une lignée tumorale de cellules humaines pancréatiques Mia-Paca2 par la 7-(2-amino-1-oxo-3-thiopropyl)-8-(cyclohexylméthyl)-2-(2-méthylphényl)-5,6,7,8-tétrahydroimidazo[1,2a]pyrazine ou la 7-(2-amino-1-oxo-3-thiopropyl)-8-(cyclohexylméthyl)-2-phényl-5,6,7,8-tétrahydroimidazo[1,2a]pyrazine (décrites dans la demande PCT WO 00/02881) en association avec différents agents anti-cancéreux.

Par convention, les produits inhibant la transduction des signaux des protéines G hétérotrimériques utilisés dans les tests seront identifiés par la lettre A, et les autres agents anti-cancéreux utilisés dans les tests par la lettre B.

### 1) Procédures

### Matériel

Les composés suivants (préparés selon les méthodes décrites précédemment) entrent dans la composition des produits testés :
- la 7-(2-amino-1-oxo-3-thiopropyl)-8-(cyclohexylméthyl)-2-(2-méthylphényl)-5,6,7,8-tétrahydroimidazo[1,2a]pyrazine (composé **A**_{**1**}) ;
- la 7-(2-amino-1-oxo-3-thiopropyl)-8-(cyclohexylméthyl)-2-phényl-5,6,7,8-tétrahydroimidazo[1,2a]pyrazine (composé **A**_{**2**}) ;
- 1-(2-(1-((4-cyano)phénylméthyl)imidazol-4-yl)-1-oxoéthyl)-2,5-dihydro-4-(2-méthoxyphényl)imidazo[1,2c][1,4]benzodiazépine (composé **B**_{**1**}) ;
- le taxol (composé **B**_{**2**}) ;
- la gemcitabine (composé **B**_{**3**}) ;
- la (±)-4-(3-chlorophényl)-6-[(4-chlorophényl)-amino-(1-méthyl-1H-imidazol-5-yl)méthyl]-1-méthyl-2(1H)quinolinone (composé **B**_{**4**}) ;
- la 4-(2-bromophényl)-1-(2-(1-((4-cyano-3-méthoxy)phénylméthyl)-imidazo-5-yl)-1-oxoéthyl)-1,2-dihydro-8-fluoro-imidazo [1,2a][1,4]-benzodiazépine (composé **B**_{**5**}).

### Lignée cellulaire

La lignée cellulaire Mia-Paca2 (cellules humaines de cancer du pancréas) a été acquise auprès de American Tissue Culture Collection (Rockville, Maryland, USA).

### Mesure de la prolifération cellulaire in vitro

Les cellules Mia-Paca2 (1500 cellules/puits) sont cultivées en plaques 96 puits précoatées par le polyhema (Sigma) qui autorise uniquement la croissance des cellules présentant un phénotype tumorigène.

Au jour 0, ces cellules sont ensemencées dans 90 µl de milieu de Eagle modifié par Dulbecco (Gibco-Brl, Cergy-Pontoise, France) complété avec 10% de sérum foetal de veau inactivé par chauffage (Gibco-Brl, Cergy-Pontoise, France ), 50000 unités/l de pénicilline et 50 mg/l streptomycine (Gibco-Brl, Cergy-Pontoise, France), et 2 mM de glutamine (Gibco-Brl, Cergy-Pontoise, France).
Les cellules ont été traitées avec des concentrations croissantes de deux produits seuls ou en association de manière matricielle, soit : au jour 1, le premier produit pendant 96 heures avec au jour 2 le second produit pendant 72 heures. Selon la méthode α, l'inhibiteur de la transduction des signaux des protéines G hétérotrimériques auquel l'agent anti-cancéreux est associé est administré *avant* ce dernier, alors que selon la méthode β, il l'est *après.*
A la fin de cette période, la quantification de la prolifération cellulaire est évaluée par test colorimétrique en se basant sur le clivage du sel de tétrazolium WST1 par les déhydrogénases mitochondriales dans les cellules vivantes conduisant à la formation de formazan (Boehringer Mannheim, Meylan, France). Ces tests sont effectués en double avec 4 déterminations pour chaque produit seul et pour chaque association testée. Ceci permet de déterminer le nombre de cellules vivantes à la fin de chaque traitement, soit la valeur observée. La valeur calculée des cellules vivantes pour chaque traitement correspond à la multiplication des valeurs observées des effets des produits séparés. Ces valeurs observées et calculées sont comparées pour chaque association. Quand la valeur observée de cellules vivantes est inférieure à la valeur calculée des cellules vivantes, une synergie est considérée. Quand la valeur observée est égale à la valeur calculée, une additivité est considérée. Quand la valeur observée est supérieure à la valeur calculée, un antagonisme est considéré.

### 2) Résultats :

Les résultats obtenus sont reportés dans les tableaux figurant ci-après.

Les résultats reportés dans les tableaux I, II, III, IV et IV montrent que les produits comprenant le composé **A**_{**1**} en association avec le composé **B**_{**1**}, le composé **B**_{**2**} ou le composé **B**_{**3**} ou ceux comprenant le composé **A**_{**2**} en association avec le composé **B**_{**4**} ou le composé **B**_{**5**} sont capables d'inhiber la prolifération *in vitro* des cellules tumorales humaines Mia-Paca2. L'effet combiné de l'association, évalué par la méthode décrite dans Cote, S. et Momparler, R.L., *Anticancer Drugs* (1993), **4**, 327-333, permet de constater une synergie pour les associations **A**_{**1**} + **B**_{**1**}, **A**_{**1**} + **B**_{**2**}**, A**_{**1**} + **B**_{**3**}**, A**_{**2**} + **B**_{**4**} et **A**_{**2**} + **B**_{**5**}.

**Tableau I**

| Doses du composé B₁ | Composé B₁ seul | *Valeurs observées (méthode α - n=4)* Composé A₁ (20 µM) + composé B₁ | *Valeurs calculées* Composé A₁ (20 µM) + composé B₁ |
|---|---|---|---|
| 0 µM | 100 | 51 ± 8,9 | |
| 0,04 µM | 94 ± 2,3 | 39 ± 8,4 | 47 ± 7,1 |
| 0,2 µM | 80 ± 4,2 | 23 ± 5,9 | 38 ± 4,9 |
| 1 µM | 68 ± 2,3 | 23 ± 6,5 | 34 ± 5,0 |

**Tableau II**

| Doses du composé B₂ | Composé B₂ seul | *Valeurs observées (méthode α - n=3)* Composé A₁ (20 µM) + composé B₂ | *Valeurs calculées* Composé A₁ (20 µM) + composé B₂ |
|---|---|---|---|
| 0 nM | 100 | 49 ± 7,7 | |
| 0,8 nM | 100 ± 5,8 | 34 ± 2,4 | 48 ± 6,7 |
| 4 nM | 100 ± 0,3 | 32 ± 2,2 | 49 ± 7,5 |
| 20 nM | 60 ± 10,6 | 17 ± 4,5 | 30 ± 7,2 |

**Tableau III**

| Doses du composé B₃ | Composé B₃ seul | *Valeurs observées (méthode α - n=3)* Composé A₁ (20 µM) + composé B₃ | *Valeurs calculées* Composé A₁ (20 µM) + composé B₃ |
|---|---|---|---|
| 0 nM | 100 | 65 ± 7,3 | |
| 4nM | 95 ± 9,8 | 48 ± 2,1 | 59 ± 1,3 |
| 20 nM | 72 ± 11,5 | 27 ± 6,8 | 45 ± 4,0 |
| 100 nM | 62 ± 1,9 | 30 ± 4,0 | 40 ± 3,1 |

**Tableau IV**

| Doses du composé B₄ | Composé B₄ seul | *Valeurs observées (méthode β- n=2)* Composé A₂ (20 µM) + composé B₄ | *Valeurs calculées* Composé A₂ (20 µM) + composé B₄ |
|---|---|---|---|
| 0 nM | 100 | 42 ± 11,0 | |
| 40 nM | 104 ± 2,0 | 28 ± 3,0 | 44 ± 12,0 |
| 0,2 µM | 90 ± 9,0 | 13 ± 2,0 | 37 ± 6,0 |
| 1 µM | 72 ± 3 | 14 ± 1,0 | 30 ± 7,0 |

**Tableau V**

| Doses du composé B₅ | Composé B₅ seul | *Valeurs observées (méthode β- n=3)* Composé A₂ (20 µM) + composé B₅ | *Valeurs calculées* Composé A₂ (20 µM) + composé B₅ |
|---|---|---|---|
| 0 µM | 100 | 44 ± 6,4 | |
| 0,2 µM | 86 ± 0,9 | 17 ± 2,7 | 38 ± 5,2 |
| 1 µM | 63 ± 2,6 | 11 ± 1,4 | 27 ± 3,0 |

## Revendications

1. Produit comprenant au moins un inhibiteur de la transduction des signaux des protéines G hétérotrimériques choisi parmi la 7-(2-amino-1-oxo-3-thiopropyl)-8-(cyclohexylméthyl)-2-(2-méthylphényl)-5,6,7,8-tétrahydroimidazo[1,2a]pyrazine, la 7-(2-amino-1-oxo-3-thiopropyl)-8-(cyclohexylméthyl)-2-phényl-5,6,7,8-tétrahydro-imidazo[1,2a]pyrazine et les sels pharmaceutiquement acceptables de ces composés et au moins un autre agent anti-cancéreux choisi parmi le groupe constitué par la 1-(2-(1-((4-cyano)phénylméthyl)imidazol-4-yl)-1-oxoéthyl)-2,5-dihydro-4-(2-méthoxyphényl)imidazo[1,2c][1,4]benzodiazépine, le taxol, la gemcitabine, la (±)-4-(3-chlorophényl)-6-[(4-chlorophényl)-amino-(1-méthyl-1H-imidazol-5-yl)méthyl]-1-méthyl-2(1H)quinolinone, la 4-(2-bromophényl)-1-(2-(1-((4-cyano-3-méthoxy)phénylméthyl)-imidazo-5-yl)-1-oxoéthyl)-1,2-dihydro-8-fluoro-imidazo[1,2a][1,4]-benzodiazépine et les sels pharmaceutiquement acceptables de ces composés, pour une utilisation thérapeutique simultanée, séparée ou étalée dans le temps, dans le traitement du cancer.

2. Produit selon la revendication 1, **caractérisé en ce que** l'inhibiteur de la transduction des signaux des protéines G hétérotrimériques est la 7-(2-amino-1-oxo-3-thiopropyl)-8-(cyclohexylméthyl)-2-(2-méthylphényl)-5,6,7,8-tétrahydroimidazo[1,2a]pyrazine et l'autre agent anti-cancéreux est la 1-(2-(1-((4-cyano)phénylméthyl)imidazol-4-yl)-1-oxoéthyl)-2,5-dihydro-4-(2-méthoxyphényl)imidazo[1,2c][1,4]benzodiazépine.

3. Produit selon la revendication 1, **caractérisé en ce que** l'inhibiteur de la transduction des signaux des protéines G hétérotrimériques est la 7-(2-amino-1-oxo-3-thiopropyl)-8-(cyclohexylméthyl)-2-(2-méthylphényl)-5,6,7,8-tétrahydroimidazo[1,2a]pyrazine et l'autre agent anti-cancéreux est le taxol.

4. Produit selon la revendication 1, **caractérisé en ce que** l'inhibiteur de la transduction des signaux des protéines G hétérotrimériques est la 7-(2-amino-1-oxo-3-thiopropyl)-8-(cyclohexylméthyl)-2-(2-méthylphényl)-5,6,7,8-tétrahydroimidazo[1,2a]pyrazine et l'autre agent anti-cancéreux est la gemcitabine.

5. Produit selon la revendication 1, **caractérisé en ce que** l'inhibiteur de la transduction des signaux des protéines G hétérotrimériques est la 7-(2-amino-1-oxo-3-thiopropyl)-8-(cyclohexylméthyl)-2-phényl-5,6,7,8-tétrahydroimidazo[1,2a]pyrazine et l'autre agent anti-cancéreux est la (±)-4-(3-chlorophényl)-6-[(4-chlorophényl)-amino-(1-méthyl-1H-imidazol-5-yl)méthyl]-1-méthyl-2(1H)quinolinone.

6. Produit selon la revendication 1, **caractérisé en ce que** l'inhibiteur de la transduction des signaux des protéines G hétérotrimériques est la 7-(2-amino-1-oxo-3-thiopropyl)-8-(cyclohexylméthyl)-2-phényl-5,6,7,8-tétrahydroimidazo[1,2a]pyrazine et l'autre agent anti-cancéreux est la 4-(2-bromophényl)-1-(2-(1-((4-cyano-3-méthoxy)phénylméthyl)-imidazo-5-yl)-1-oxoéthyl)-1,2-dihydro-8-fluoro-imidazo [1,2a][1,4]-benzodiazépine.

## Patentansprüche

1. Produkt, das mindestens einen Hemmer der Transduktion der Signale der heterotrimeren G-Proteine, der aus 7-(2-Amino-1-oxo-3-thiopropyl)-8-(cyclohexylmethyl)-2-(2-methylphenyl)-5,6,7,8-tetrahydroimidazo[1,2a]pyrazin, 7-(2-Amino-1-oxo-3-thiopropyl)-8-(cyclohexylmethyl)-2-phenyl-5,6,7,8-tetrahydroimidazo[1,2a]pyrazin und den pharmazeutisch verträglichen Salzen dieser Verbindungen ausgewählt ist, und mindestens ein anderes anticancerös wirkendes Mittel enthält, das aus der Gruppe ausgewählt ist, die aus 1-(2-(1-((4-Cyan)phenylmethyl)imidazol-4-yl)-1-oxoethyl)-2,5-dihydro-4-(2-methoxyphenyl)imidazo[1,2c][1,4]benzodiazepin, Taxol, Gemcitabin, (±)-4-(3-Chlorphenyl)-6-[(4-chlorphenyl) -amino- (1-methyl-1H-imidazol-5-yl)methyl]-1-methyl-2(1H)chinolinon, 4-(2-Bromphenyl)-1-(2-(1-((4-cyan-3-methoxy)phenylmethyl)-imidazo-5-yl)-1-oxoethyl)-1,2-dihydro-8-fluorimidazo[1,2a][1,4]-benzodiazepin und den pharmazeutisch verträglichen Salzen dieser Verbindungen besteht, für eine gleichzeitige, getrennte oder zeitlich verteilte therapeutische Verwendung in der Krebsbehandlung.

2. Produkt nach Anspruch 1, **dadurch gekennzeichnet, daß** der Hemmer der Transduktion der Signale der heterotrimeren G-Proteine 7-(2-Amino-1-oxo-3-thiopropyl)-8-(cyclohexylmethyl)-2-(2-methylphenyl)-5,6,7,8-tetrahydroimidazo [1,2a]pyrazin ist und das andere anticancerös wirkende Mittel 1-(2-(1-((4-Cyan)phenylmethyl)imidazol-4-yl)-1-oxoethyl)-2,5-dihydro-4-(2-methoxyphenyl)imidazo[1,2c][1,4] benzodiazepin ist.

3. Produkt nach Anspruch 1, **dadurch gekennzeichnet, daß** der Hemmer der Transduktion der Signale der heterotrimeren G-Proteine 7-(2-Amino-1-oxo-3-thiopropyl)-8-(cyclohexylmethyl)-2-(2-methylphenyl)-5,6,7,8-tetrahydroimidazo [1,2a]pyrazin ist und das andere anticancerös wirkende Mittel Taxol ist.

4. Produkt nach Anspruch 1, **dadurch gekennzeichnet, daß** der Hemmer der Transduktion der Signale der heterotrimeren G-Proteine 7-(2-Amino-1-oxo-3-thiopropyl)-8-(cyclohexylmethyl)-2-(2-methylphenyl)-5,6,7,8-tetrahydroimidazo [1,2a]pyrazin ist und das andere anticancerös wirkende Mittel Gemcitabin ist.

5. Produkt nach Anspruch 1, **dadurch gekennzeichnet, daß** der Hemmer der Transduktion der Signale der heterotrimeren G-Proteine 7-(2-Amino-1-oxo-3-thiopropyl)-8-(cyclohexylmethyl)-2-phenyl-5,6,7,8-tetrahydroimidazo[1,2a]pyrazin ist und das andere anticancerös wirkende Mittel (±)-4-(3-Chlorphenyl)-6-[(4-chlorphenyl)-amino-(1-methyl-1H-imidazol-5-yl)methyl]-1-methyl-2(1H)chinolinon ist.

6. Produkt nach Anspruch 1, **dadurch gekennzeichnet, daß** der Hemmer der Transduktion der Signale der heterotrimeren G-Proteine 7-(2-Amino-1-oxo-3-thiopropyl)-8-(cyclohexylmethyl)-2-phenyl-5,6,7,8-tetrahydroimidazo[1,2a]pyrazin ist und das andere anticancerös wirkende Mittel 4-(2-Bromphenyl)-1-(2-(1-((4-cyan-3-methoxy)phenylmethyl)-imidazo-5-yl)-1-oxoethyl)-1,2-dihydro-8-fluorimidazo[1,2a][1,4]-benzodiazepin ist.

## Claims

1. Product comprising at least one transduction inhibitor of heterotrimeric G protein signals chosen from 7-(2-amino-1-oxo-3-thiopropyl)-8-(cyclohexylmethyl)-2-(2-methylphenyl)-5,6,7,8-tetrahydroimidazo[1,2a]pyrazine, 7-(2-amino-1-oxo-3-thiopropyl)-8-(cyclohexylmethyl)-2-phenyl-5,6,7,8-tetrahydroimidazo[1,2a]pyrazine and the pharmaceutically acceptable salts of these compounds and at least one other anti-cancer agent chosen from the group constituted by 1-(2-(1-((4-cyano)phenylmethyl) imidazol-4-yl)-1-oxoethyl-2,5)-dihydro-4-(2-methoxyphenyl)imidazo[1,2c] [1,4]benzodiazepine, taxol, gemcitabine, (±)-4-(3-chlorophenyl)-6-[(4-chlorophenyl)-amino-(1-methyl-1H-imidazol-5-yl)methyl]-1-methyl-2(1H)quinolinone, 4-(2-bromophenyl)-1-(2-(1-((4-cyano-3-methoxy)phenylmethyl)-imidazo-5-yl)-1-oxoethyl)-1,2-dihydro-8-fluoro-imidazo[1,2a][1,4]-benzodiazepine, and the pharmaceutically acceptable salts of these compounds, for a therapeutic use simultaneously, separately or spread over a period of time, in the treatment of the cancer.

2. Product according to claim 1, **characterized in that** the transduction inhibitor of heterotrimeric G protein signals is 7-(2-amino-1-oxo-3-thiopropyl)-8-(cyclohexylmethyl)-2-(2-methylphenyl)-5,6,7,8-tetrahydroimidazo[1,2a]pyrazine, and the other anti-cancer agent is 1-(2-(1-((4-cyano)phenylmethyl)imidazol-4-yl)-1-oxoethyl)-2,5-dihydro-4-(2-methoxyphenyl)imidazo[1,2c][1,4]benzodiazepine.

3. Product according to claim 2, **characterized in that** the transduction inhibitor of heterotrimeric G protein signals is 7-(2-amino-1-oxo-3-thiopropyl)-8-(cyclohexylmethyl)-2-(2-methylphenyl)-5,6,7,8-tetrahydroimidazo[1,2a]pyrazine and the other anti-cancer agent is taxol.

4. Product according to claim 1, **characterized in that** the transduction inhibitor of heterotrimeric G protein signals is 7-(2-amino-1-oxo-3-thiopropyl)-8-(cyclohexylmethyl)-2-(2-methylphenyl)-5,6,7,8-tetrahydroimidazo[1,2a]pyrazine and the other anti-cancer agent is gemcitabine.

5. Product according to claim 1, **characterized in that** the transduction inhibitor of heterotrimeric G protein signals is 7-(2-amino-1-oxo-3-thiopropyl)-8-(cyclohexylmethyl)-2-phenyl-5,6,7,8-tetrahydroimidazo[1,2a]pyrazine and the other anti-cancer agent is (±)-4-(3-chlorophenyl)-6-[(4-chlorophenyl)-amino-(1-methyl-1H-imidazol-5-yl)methyl]-1-methyl-2(1H)quinolinone.

6. Product according to claim 1, **characterized in that** the transduction inhibitor of heterotrimeric G protein signals is 7-(2-amino-1-oxo-3-thiopropyl)-8-(cyclohexylmethyl)-2-phenyl-5,6,7,8-tetrahydroimidazo[1,2a]pyrazine and the other anti-cancer agent is 4-(2-bromophenyl)-1-(2-(1-((4-cyano-3-methoxy)phenylmethyl)-imidazo-5-yl)-1-oxoethyl)-1,2-dihydro-8-fluoro-imidazo[1,2a][1,4]-benzodiazepine.
